# EUROPEAN PATENT SPECIFICATION

(11) **EP 3 663 394 B1**
(45) Date of publication and mention of the grant of the patent: **05.10.2022**
(21) Application number: 18923225.9
(22) Date of filing: 19.06.2018
(51) Int. Cl.: C12N 5/0775, A61K 35/28, A61P 9/10, A61P 9/00

(54) **METHOD FOR PREPARING MESENCHYMAL STEM CELL-DERIVED EXOSOMES ON BASIS OF DRUG PRETREATMENT**
VERFAHREN ZUR HERSTELLUNG VON EXOSOMEN AUS MESENCHYMALEN STAMMZELLEN AUF BASIS VON ARZNEIVORBEHANDLUNG
PROCÉDÉ POUR LA PRÉPARATION D'EXOSOMES DÉRIVÉS DE CELLULES SOUCHES MÉSENCHYMATEUSES BASÉ SUR UN PRÉTRAITEMENT AVEC UN MÉDICAMENT

(43) Date of publication of application: 10.06.2020
(73) Proprietor: Fuwai Hospital, Chinese Academy Of Medical Sciences And Peking Union Medical College, Beijing 100037 (CN)
(72) Inventor: YANG, Yuejin, Beijing 100037 (CN); HUANG, Peisen, Beijing 100037 (CN); CHEN, Guihao, Beijing 100037 (CN)
(74) Representative: Hoffmann Eitle
(86) International application number: PCT/CN2018/091843
(87) International publication number: WO 2019/241910

(56) References cited:
- CN-A- 101 129 356
- CN-A- 106 282 107
- CN-A- 107 137 700
- CN-A- 108 728 410
- US-A1- 2017 319 504
- HUANG PEISEN ET AL: "ATORVASTATIN IMPROVES THE THERAPEUTIC EFFICACY OF MESENCHYMAL STEM CELLS DERIVED EXOSOMES IN ACUTE MYOCARDIAL INFARCTION BY PROMOTING ENDOTHELIAL CELL FUNCTION", JOURNAL OF THE AMERICAN COLLEGE OF CARDIOLOGY SUPPLEMENT, vol. 71, no. 11, 10 March 2018 (2018-03-10), page A83, XP055763526, DOI: doi:10.1016/S0735-1097(18)30624-7
- DAVOD PASHOUTAN SARVAR ET AL: "Mesenchymal Stem Cell-Derived Exosomes: New Opportunity in Cell-Free Therapy", ADVANCED PHARMACEUTICAL BULLETIN, vol. 6, no. 3, 25 September 2016 (2016-09-25), pages 293-299, XP055582068, ISSN: 2228-5881, DOI: 10.15171/apb.2016.041
- Na Li ET AL: "Intravenous administration of atorvastatin-pretreated mesenchymal stem cells improves cardiac performance after acute myocardial infarction: role of CXCR4", Am J Transl Res, 15 June 2015 (2015-06-15), pages 1058-1070, XP055764267, ISSN: 1943-8141 Retrieved from the Internet: URL:http://www.ajtr.org/files/ajtr0009781. pdf [retrieved on 2021-01-13]
- LAMPARSKI H G ET AL: "Production and characterization of clinical grade exosomes derived from dendritic cells", JOURNAL OF IMMUNOLOGICAL METHODS, ELSEVIER SCIENCE PUBLISHERS B.V.,AMSTERDAM, NL, vol. 270, no. 2, 15 December 2002 (2002-12-15), pages 211-226, XP004387999, ISSN: 0022-1759
- RUENN CHAI LAI ET AL: "Exosome secreted by MSC reduces myocardial ischemia/reperfusion injury", STEM CELL RESEARCH, vol. 4, no. 3, 1 May 2010 (2010-05-01), pages 214-222, XP055555858, NL ISSN: 1873-5061, DOI: 10.1016/j.scr.2009.12.003
- HUANG PEISEN ET AL: "New strategies for improving stem cell therapy in ischemic heart disease", HEART FAILURE REVIEWS, KLUWER ACADEMIC PUBLISHERS, DORDRECHT, NL, vol. 21, no. 6, 26 July 2016 (2016-07-26), pages 737-752, XP036085165, ISSN: 1382-4147, DOI: 10.1007/S10741-016-9576-1 [retrieved on 2016-07-26]

## Description

### Technical field

The invention relates to a preparation method of mesenchymal stem cell-derived exosomes based on drug pretreatment, specifically, an efficient preparation method of mesenchymal stem cell-derived exosomes based on statins pretreatment.

### Background

WHO statistics show that cardiovascular diseases were the leading cause of death in the world in 2016 (about 17.6 million, 32.2%), among which 9.48 million died of ischemic heart disease (17.3%) (Collaborators GM. Global, regional, and national under-5 mortality, adult mortality, age-specific mortality, and life expectancy, 1970-2016: a systematic analysis for the Global Burden of Disease Study 2016. The Lancet 2017; 390(10100):1084-1150; Collaborators GCOD. Global, regional, and national age-sex specific mortality for 264 causes of death, 1980-2016: a systematic analysis for the Global Burden of Disease Study 2016. The Lancet 2017; 390(10100):1151-1210). Acute myocardial infarction (AMI) causes massive myocardial ischemic necrosis, and the replacement by scar tissues in turn leads to heart failure and death. Current therapeutic approaches cannot effectively regenerate and repair myocardium. In recent years, high expectation has been given to stem cell transplantation, especially mesenchymal stem cell (MSCs) transplantation such as bone marrow-mesenchymal stem cells (BM-MSCs) transplantation, in the treatment of AMI (Orlic D, Kajstura J, Chimenti S, Jakoniuk I, Anderson SM, Li B, Pickel J, McKay R, Nadal-Ginard B, Bodine DM and others. Bone marrow cells regenerate infarcted myocardium. Nature 2001;410(6829):701-705; Fisher SA, Doree C, Mathur A, Martin-Rendon E. Meta-Analysis of Cell Therapy Trials for Patients With Heart Failure. Circulation Research 2015;116(8):1361-1377; Afzal MR, Samanta A, Shah ZI, Jeevanantham V, Abdel-Latif A, Zuba-Surma EK, Dawn B. Adult Bone Marrow Cell Therapy for Ischemic Heart Disease: Evidence and Insights From Randomized Controlled Trials. Circ Res 2015; 117(6):558-575). However, clinical studies have shown that mesenchymal stem cells could improve cardiac function after infarction to a certain extent through paracrine protection, although this effect is hardly noticable. Further studies have shown that the primary mechanism underlying the paracrine protection is by secretion of an extracellular vesicles, i.e., exosomes (Makridakis M, Roubelakis MG, Vlahou A. Stem cells: insights into the secretome. Biochim Biophys Acta 2013;1834(11):2380-2384; Huang P, Tian X, Li Q, Yang Y. New strategies for improving stem cell therapy in ischemic heart disease. Heart Fail Rev 2016;21(6):737-752; Lai RC, Arslan F, Lee MM, Sze NS, Choo A, Chen TS, Salto-Tellez M, Timmers L, Lee CN, El OR and others. Exosome secreted by MSC reduces myocardial ischemia/reperfusion injury. Stem Cell Res 2010; 4(3):214-222). Exosomes is advantageous in its rich sources and stability, with no immunogenicity, and BM-MSC-derived exosomes (MSC-Exo) can improve the myocardial infarction microenvironment. Thus, it is a promising next-generation myocardial repair product (Lamichhane TN, Sokic S, Schardt JS, Raiker RS, Lin JW, Jay SM. Emerging Roles for Extracellular Vesicles in Tissue Engineering and Regenerative Medicine. Tissue Engineering Part B: Reviews 2015; 21(1):45-54).

### Summary of the Invention

An object of the present invention is to provide a preparation method of mesenchymal stem cell-derived exosomes with cardioprotective ability.

According to the study in the present invention, it has been discovered that pretreatment of mesenchymal stem cells with atorvastatin (ATV) can significantly improve the anti-apoptosis ability and homing ability of mesenchymal stem cells, and produce stem cell-derived exosomes with abilities of significantly improving myocardial infarction microenvironment and highly efficient myocardial repairing as further defined in the claims.

In one aspect, the present invention provides the preparation method of the claims.

According to a specific embodiment of the present invention, the preparation method is a method of bone marrow mesenchymal stem cell-derived exosomes with an increased level of IncRNA H19 expression, including: adding 1 µM atorvastatin into a medium of the mesenchymal stem cells for pretreatment for 24 hours, and then replacing the cell culture medium with a exosome-free medium for continued culturing;
after 48 hours, collecting the conditioned medium and isolating exosomes derived from the mesenchymal stem cells pretreated with statins by repeated ultracentrifugation; wherein the exosomes are spherical or disc shaped with a particle size distribution in the range of 30-150 nm, and highly express IncRNA H19 by up to 10 times or more compared exosomes secreted by BM-MSCs without pre-treatment.

According to a specific embodiment of the present invention, in the preparation method of mesenchymal stem cell-derived exosomes of the invention, the ultracentrifugation process includes the steps of:
after collecting the conditioned medium, successively removing cells by centrifugation, cell debris by centrifugation, and large vesicles by high speed centrifugation, and then collecting the pellet by ultracentrifugation and re-suspending, and conducting further ultracentrifugation to obtain the exosome.

In one preferred specific embodiment of the present invention, the ultracentrifugation process includes steps of: after collecting the conditioned medium, removing cells by centrifugation at 300 g for 10 minutes, removing cell debris by centrifugation at 2,000 g for 20 minutes; removing large vesicles by high speed centrifugation at 16,500 g for 30 minutes; collecting the pellet by ultracentrifugation at 120,000 g for 70 minutes and re-suspending, and conducting further ultracentrifugation at 120,000 g for 70 minutes to obtain the exosomes.

In a reference aspect, not part of the invention, the present application also provides exosomes prepared by the preparation method as described in the invention.

In still another reference the present specification also provides use of statins in the preparation of a formulation that promote the anti-apoptotic and/or homing ability of mesenchymal stem cells.

In yet another reference, the present specification also provides use of statins in the preparation of a formulation that promote the secretion of exosomes having abilities of improving the myocardial infarction microenvironment and/or myocardial repairing from mesenchymal stem cells.

According to the present invention, the statin is atorvastatin; mesenchymal stem cells were pretreated with 1 µM atorvastatin for 24 hours.

According to the invention, the mesenchymal stem cells are bone marrow mesenchymal stem cells.

In the present invention, exosomes capable of effective myocardial repairing and endothelial protection are obtained by pretreatment of BM-MSC with 1 µM ATV for 24 hours.

### Brief Description of the Drawings

Figs. 1A-1C show the identification results of the mesenchymal stem cell-derived exosome in an example of the present invention.
Figs. 2A-2D show the difference in the effect of exosomes derived from mesenchymal stem cells pretreated with ATV at different concentrations on endothelial cells.
Figs. 3A-3H show the test results of tube formation, migration and survival of vascular endothelial cells promoted by exosomes derived from ATV pretreated mesenchymal stem cells.
Figs. 4A-4F show the test results in which exosomes derived from mesenchymal stem cells pretreated with ATV significantly improve cardiac function and reduce myocardial infarction area after myocardial infarction in rats.
Figs. 5A-5K show the test results in which the protective effect of exosome derived from mesenchymal stem cell pretreated with ATV is related to its up-regulation of IncRNA H19.

### Detailed Description

The present invention will be further explained with respect to its features and technical effects by means of the specific examples below, but the invention is not limited thereto in any way. In the Examples, all raw reagents and materials are commercially available. Experimental procedures with unspecified conditions are conventional procedures and conventional conditions well known in the related art, or in accordance with the conditions recommended by the instrument manufacturer.

### Example 1

Preparation of mesenchymal stem cell-derived exosomes: BM-MSCs of rats (Sprague-Dawley rats, 60-80 g) was isolated by differential adhesion and amplified via passage to the third-fourth generation for use. After 24 hours of pretreatment with ATV in BM-MSC culture medium (IMDM, Invitrogen, US), the cell culture medium was replaced with exosome free FBS containing medium (IMDM medium containing 10% FBS after 18 hours of ultracentrifugation) for continued culturing. After 48 hours, the conditioned medium was collected and exosomes secreted by BM-MSCs pretreated with ATV (MSC^{ATV}-Exo) were isolated and obtained by ultracentrifugation. Specifically, the ultracentrifugation process included the steps of: after collecting the conditioned medium, removing cells by centrifugation at 300 g for 10 minutes, and removing cell debris by centrifugation at 2,000 g for 20 minutes; removing macrovesicles by high speed centrifugation at 16,500 g for 30 minutes; collecting the pellet by ultracentrifugation at 120,000 g for 70 minutes and re-suspending, and conducting further ultracentrifugation at 120,000 g for 70 minutes to obtain the exosome.

Identification of the prepared MSC^{ATV}-Exo: including electron microscopy (HITACHI, H-600IV, Japan) analysis of morphology and structures, NTA (Malvern Instruments, NanoSight, UK) analysis of particle size distribution of exosomes, and Western Blot assaying of exosome protein markers.

The effects of pretreatment with ATV at different concentrations on MSC^{ATV}-Exo function were compared, and the optimum ATV concentration for the pretreatment was selected. Functionality evaluation of MSC^{ATV}-Exo pretreated at this optimal ATV concentration was carried out, including the impact on tube formation, migration and anti-apoptosis of vascular endothelial cells, and the effect in improving cardiac function and reducing myocardial infarction area after myocardial infarction in rats upon intramyocardial injection. Finally, molecular biological evaluation, i.e., assay of the IncRNA H19 expression level in MSC^{ATV}-Exo, was conducted.

### Evaluation Indicators (Research Results)

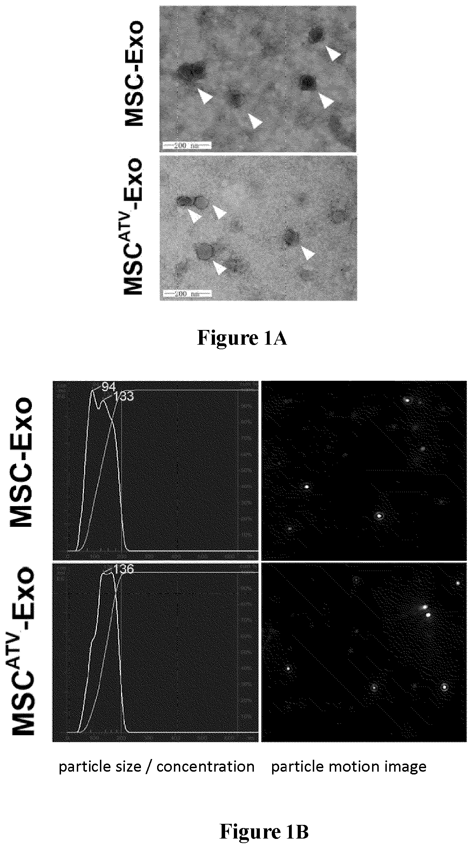

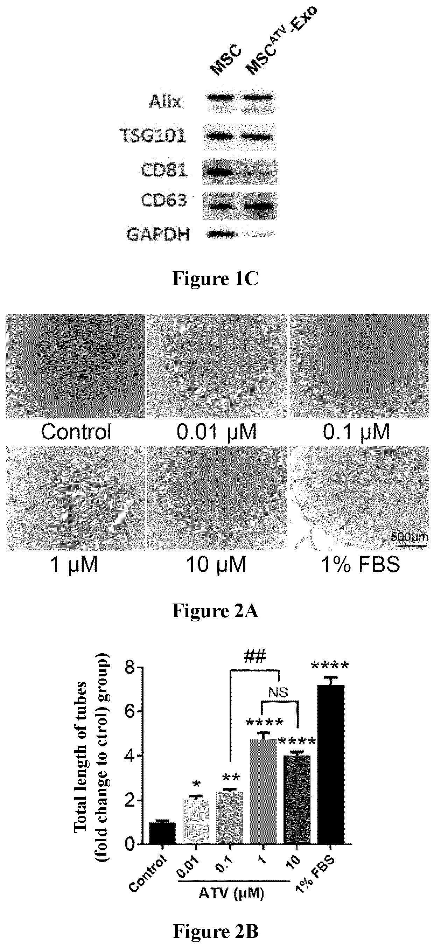

The MSC^{ATV}-Exo obtained by ultracentrifugation is spherical or disc shaped under the electron microscope, having a size of about 100 nm. NTA analysis shows a particle size distribution in the range of 30-150 nm. Western Blot assay shows high expression of exosome protein markers such as TSG101, Alix, CD63, and CD81 in MSC^{ATV}-Exo. No significant difference is evident in morphology, particle size distribution, and protein markers between the exosomes secreted by BM-MSCs pretreated with ATV or without pretreatment. Detailed results are demonstrated in Fig. 1A to Fig. 1C, and in Fig. 1A: the morphological structure of mesenchymal stem cell-derived exosomes (MSC-Exo) in a spherical or disc shape with a size of about 100 nm are observed under electron microscope, which is not changed after statins pretreatment; in Fig. 1B: the particle size distribution of MSC-Exo was analyzed by NTA, where both the MSC-Exo pretreated with statins and those without any pretreatment have a particle size distribution in the range of 30-150 nm; and in Fig. 1C: exosome protein markers are identified, where exosome protein markers such as TSG101, Alix, CD63, and CD81 were highly expressed in MSC-Exo pretreated with statins.

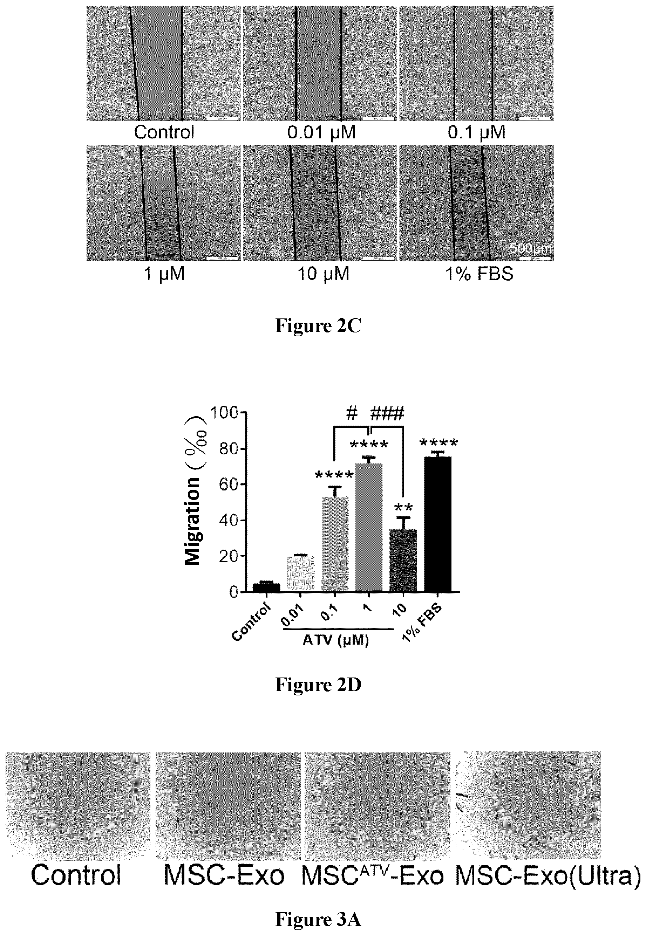

MSC^{ATV}-Exo obtained by pretreatment with ATV at different concentrations (0.01, 0.1, 1, 10 µM) were subjected to functionality analysis, in which it was found that MSC^{ATV}-Exo pretreated with 1 µM ATV had the most prominent effect on promoting tube formation and migration of endothelial cells. Detailed results are demonstrated in Fig. 2A to Fig. 2D, and in Fig. 2A to Fig. 2B: the effects of exosomes extracted from mesenchymal stem cells pretreated with ATV at different concentrations (0.01, 0.1, 1, 10 µM) on tube formation are compared, among which the 1 µM ATV pretreatment had the best effect (Fig. 2B); in Fig. 2C to Fig. 2D: the effects of exosomes extracted from mesenchymal stem cells pretreated with ATV at different concentrations on tube formation were compared, among which the 1 µM ATV pretreatment had the best effect (Fig. 2D).

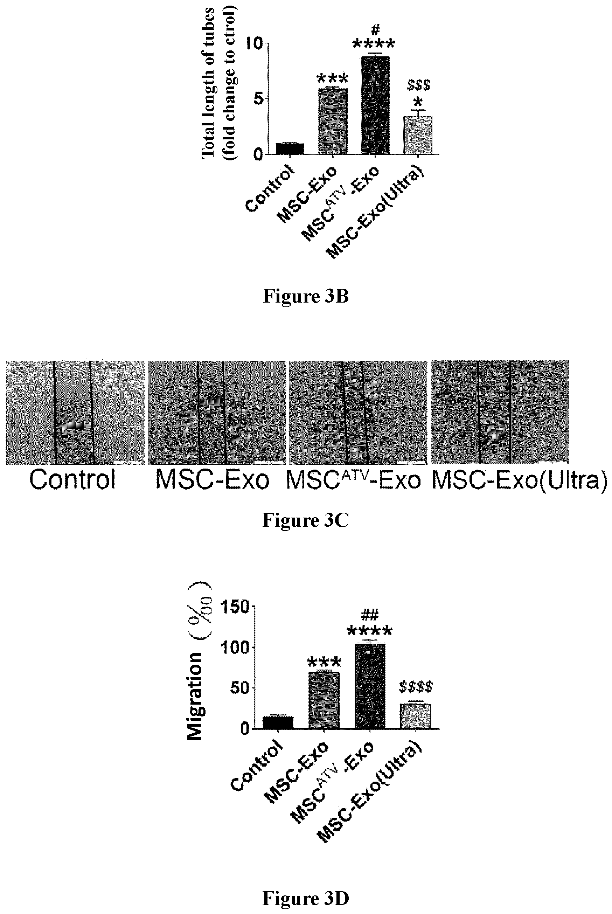

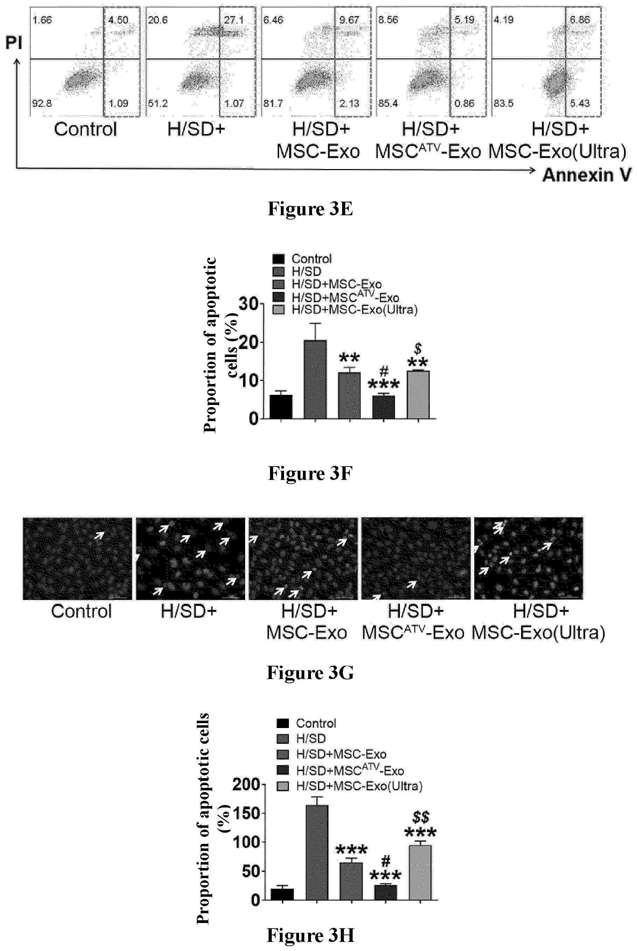

As compared to MSC-Exo without ATV pretreatment, MSC^{ATV}-Exo can significantly promote the tube formation and migration of endothelial cells, and promote the survival and anti-apoptosis of endothelial cells under hypoxia and serum deprived conditions. Detailed results are demonstrated in Fig. 3A to Fig. 3H, and Fig. 3A to Fig. 3B: in tube formation tests, the exosome derived from mesenchymal stem cells pretreated with ATV (MSC^{ATV}-Exo) significantly promotes tube formation of endothelial cells; in Fig. 3C to Fig. 3D: in migration tests, MSC^{ATV}-Exo significantly promotes the migration of endothelial cells as compared to the control group; in Fig. 3E to Fig. 3F: in flow cytometry assay, MSC^{ATV}-Exo significantly promotes the survival of endothelial cells under hypoxia and serum deprived conditions as compared to the control group; and in Fig. 3G to Fig. 3H: with Hoechst 33342 staining, MSC^{ATV}-Exo significantly reduces apoptosis of endothelial cells under hypoxia and serum deprived conditions as compared to the control group.

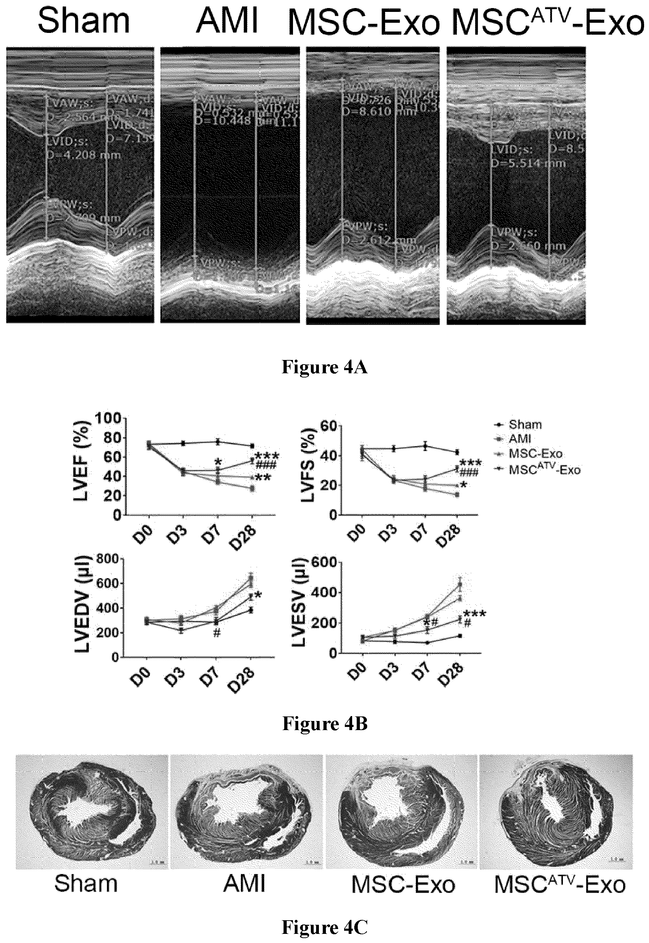

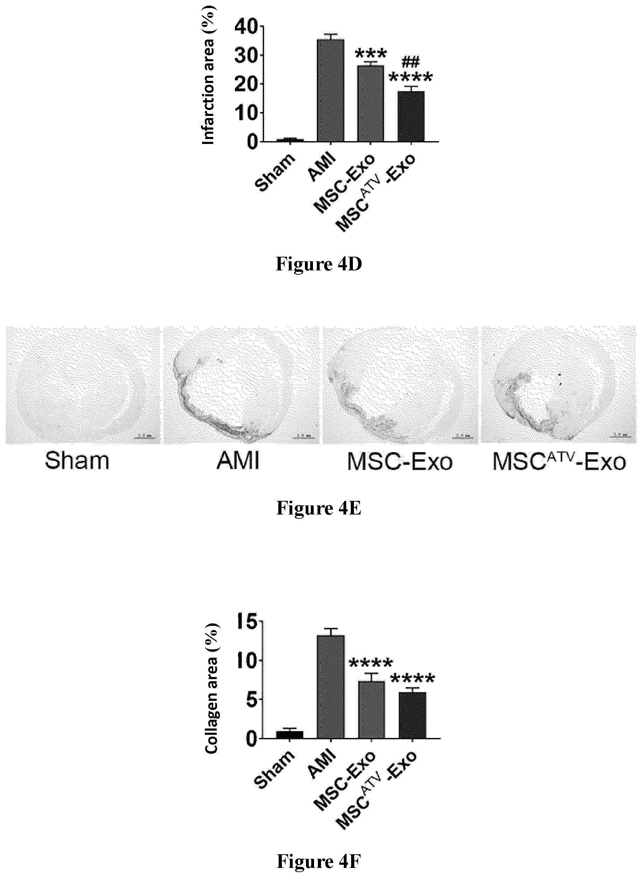

As compared to MSC-Exo without ATV pretreatment, upon its intramyocardial injection, MSC^{ATV}-Exo can significantly improve cardiac function and reduce myocardial infarction area after myocardial infarction in rats. Detailed results are demonstrated in Fig. 4A to Fig. 4F, and in Fig. 4A to Fig. 4B: transplantation of exosomes derived from mesenchymal stem cells pretreated with ATV (MSC^{ATV}-Exo) significantly improves the cardiac function in rats after myocardial infarction; in Fig. 4C to Fig. 4D: Masson staining shows that transplantation of MSC^{ATV}-Exo significantly reduces myocardial infarction area in rats; and Fig. 4E to Fig. 4F: Sirius red staining suggests that MSC^{ATV}-Exo transplantation remarkably reduce the local collagen deposition in rats after myocardial infarction.

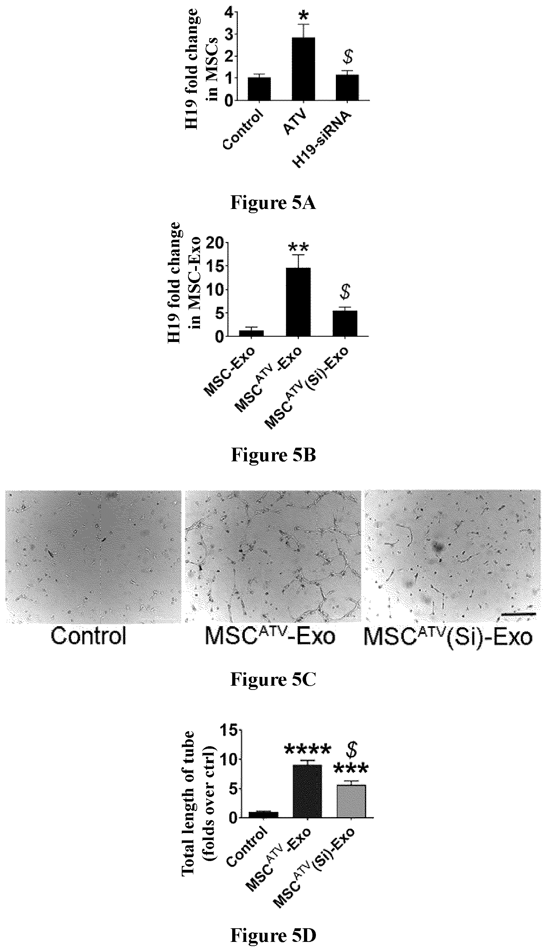

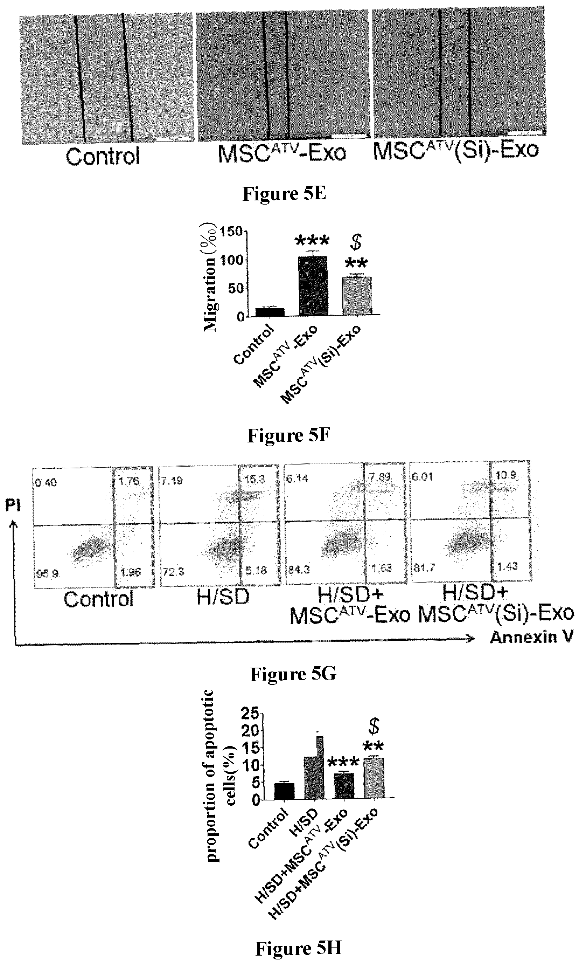

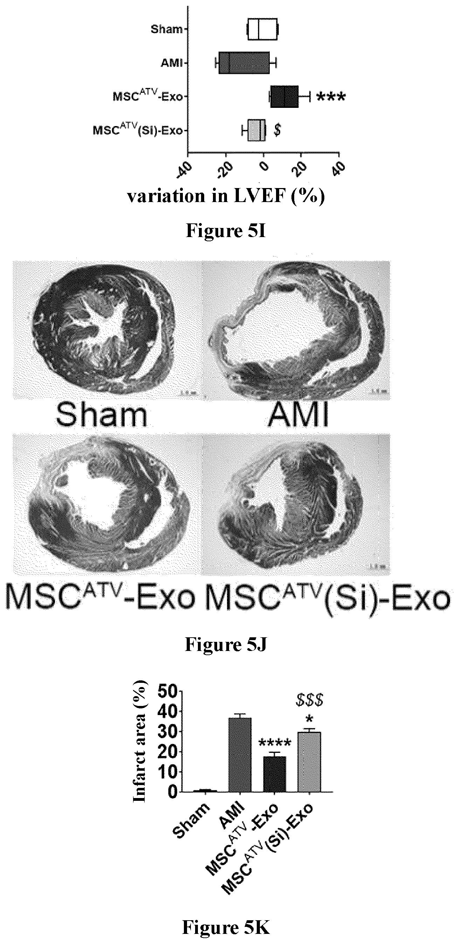

As compared to MSC-Exo, MSC^{ATV}-Exo highly expresses IncRNA H19 by up to 10 times or more. after the expression level of IncRNA H19 in MSC pretreated with ATV was knocked down by small interfering RNA, the exosome secreted thereby (MSC^{ATV}(Si)-Exo) was extracted, with the above discussed protective effects eliminated, suggesting that IncRNA H19 was related to the efficacy of MSC^{ATV}-Exo in endothelial cell protection, cardiac function improvement, and myocardial infarction area reduction. Detailed results are demonstrated in Fig. 5A to Fig. 5K, and in Fig. 5A to Fig. 5B: ATV pretreated mesenchymal stem cell-derived exosome (MSC^{ATV}-Exo) highly expresses IncRNA H19, whereas the expression of IncRNA H19 significantly decreased in the exosome with small interfering RNA knockdown (MSC^{ATV}(Si)-Exo); in Fig. 5C to Fig. 5H: in comparison to MSC^{ATV}-Exo, MSC^{ATV}(Si)-Exo has a lesser endothelial protective effect; and Fig. 5I to Fig. 5K: in comparison to MSC^{ATV}-Exo, MSC^{ATV}(Si)-Exo has lesser effects in improving cardiac function and repairing myocardium after infarction.

**Conclusion:** Exosomes capable of effective endothelial protection and myocardial repairing can be obtained by pretreatment of BM-MSC with 1 µM ATV for 24 hours, and the mechanism thereof is related to the up-regulation of IncRNA H19 in the exosomes.

## Claims

1. A preparation method of bone marrow mesenchymal stem cell-derived exosomes with an increased level of IncRNA H19 expression, including: adding 1 µM atorvastatin into a medium of the mesenchymal stem cells for pretreatment for 24 hours, and then replacing the cell culture medium with a exosome-free medium for continued culturing;
after 48 hours, collecting the conditioned medium and isolating exosomes derived from the mesenchymal stem cells pretreated with statins by repeated ultracentrifugation;
wherein the exosomes are spherical or disc shaped with a particle size distribution in the range of 30-150 nm, and highly express IncRNA H19 by up to 10 times or more compared exosomes secreted by BM-MSCs without pre-treatment.

2. The method according to claim 1, wherein the ultracentrifugation process includes the steps of:
after collecting the conditioned medium, removing cells by centrifugation at 300 g for 10 minutes, removing cell debris by centrifugation at 2,000 g for 20 minutes; removing large vesicles by high speed centrifugation at 16,500 g for 30 minutes; collecting the pellet by ultracentrifugation at 120,000 g for 70 minutes and re-suspending, and conducting further ultracentrifugation at 120,000 g for 70 minutes to obtain the exosome.

## Patentansprüche

1. Zubereitungsverfahren von aus mesenchymalen Stammzellen des Knochenmarks gewonnenen Exosomen mit einer erhöhten Expression von lncRNA H19, einschließend: Hinzufügen von 1 µM Atorvastatin zu einem Medium der mesenchymalen Stammzellen zur Vorbehandlung für 24 Stunden, und dann Ersetzen des Zellkulturmediums mit einem Exosom-freien Medium für fortgesetztes Kultivieren;
nach 48 Stunden, Sammeln des konditionierten Mediums und Isolieren von Exosomen, die von den mit Statinen vorbehandelten mesenchymalen Stammzellen gewonnen wurden, durch wiederholte Ultrazentrifugation;
wobei die Exosomen kugelförmig oder scheibenförmig mit einer Partikelgrößenverteilung in dem Bereich von 30-150 nm sind, und lncRNA H19 hochgradig exprimieren um bis zu 10-mal oder mehr verglichen mit sezernierten Exosomen von BM-MSCs ohne Vorbehandlung.

2. Verfahren gemäß Anspruch 1, wobei der Ultrazentrifugationsvorgang die folgenden Schritte einschließt:
nach dem Sammeln des konditionierten Mediums, Entfernen von Zellen durch Zentrifugation bei 300 g für 10 Minuten, Entfernen von Zelltrümmern durch Zentrifugation bei 2.000 g für 20 Minuten; Entfernen von großen Vesikeln durch Hochgeschwindigkeitszentrifugation bei 16.500 g für 30 Minuten; Sammeln des Pellets durch Ultrazentrifugation bei 120.000 g für 70 Minuten und Resuspendieren, und Durchführen weiterer Ultrazentrifugation bei 120.000 g für 70 Minuten, um das Exosom zu erhalten.

## Revendications

1. Procédé de préparation d'exosomes dérivés de cellules souches mésenchymateuses de la moelle osseuse avec une augmentation du taux d'expression des ARNlnc H19, incluant: l'ajout de 1 µM d'atorvastatine dans un milieu des cellules souches mésenchymateuses pour le prétraitement pendant 24 heures, et ensuite le remplacement du milieu de culture cellulaire par un milieu exempt d'exosome pour une culture continue ;
après 48 heures, le recueil du milieu conditionné et l'isolement des exosomes dérivés des cellules souches mésenchymateuses prétraitées par des statines par ultracentrifugation répétée ;
dans lequel les exosomes sont sphériques ou en forme de disque avec une distribution granulométrique située dans la plage 30-150 mm, et expriment fortement l'ARNlnc H19 pouvant atteindre jusqu'à 10 fois ou plus par rapport aux exosomes sécrétés par les BM-MSC sans prétraitement.

2. Procédé selon la revendication 1, dans lequel la méthode d'ultracentrifugation inclut les étapes consistant à :
après le recueil du milieu conditionné, éliminer les cellules par centrifugation à 300 g pendant 10 minutes, éliminer les débris cellulaires par centrifugation à 2000 g pendant 20 minutes ; éliminer les grandes vésicules par centrifugation à grande vitesse à 16 500 g pendant 30 minutes; recueillir la pastille par ultracentrifugation à 120 000 g pendant 70 minutes et remettre en suspension, et réaliser en outre une ultracentrifugation à 120 000 g pendant 70 minutes pour obtenir l'exosome.
